(19) Europäisches Patentamt

European Patent Office

Office européen des brevets

(11) **EP 1 203 572 B1**

(12) **EUROPÄISCHE PATENTSCHRIFT**

(45) Veröffentlichungstag und Bekanntmachung des Hinweises auf die Patenterteilung:
**23.11.2005 Patentblatt 2005/47**

(51) Int Cl.⁷: **A61F 13/02**, C09J 7/02

(21) Anmeldenummer: **01122414.4**

(22) Anmeldetag: **20.09.2001**

(54) **Verfahren zur Herstellung von merhschichtigen dünnen Pflastern mit einem Trägermaterial auf Polyurethanbasis**

Method of production of multilayer plaster having a polyurethane-based carrier layer

Méthode de fabrication d'un pansement multicouche sur une couche de support à base de polyuréthane

(84) Benannte Vertragsstaaten:
**AT BE CH CY DE DK ES FI FR GB GR IE IT LI LU MC NL PT SE TR**

(30) Priorität: **22.09.2000 DE 10047700**

(43) Veröffentlichungstag der Anmeldung:
**08.05.2002 Patentblatt 2002/19**

(73) Patentinhaber: **Beiersdorf AG**
**20245 Hamburg (DE)**

(72) Erfinder:
• **Kartheus, Holger**
**20255 Hamburg (DE)**
• **Quandt, Jürgen Christian**
**25336 Klein Nordende (DE)**
• **Beckmann, Andreas**
**22609 Hamburg (DE)**
• **Sachau, Günther**
**25451 Quickborn (DE)**
• **Ruhnau, Klaus, Dr.**
**22885 Barsbüttel (DE)**
• **Möller, Thomas**
**22589 Hamburg (DE)**

(56) Entgegenhaltungen:
WO-A-94/07935          DE-A- 4 308 445
DE-A- 19 826 592          US-A- 5 935 363

**Beschreibung**

Die Erfindung betrifft ein Verfahren zur Herstellung von mehrschichtigen dünnen Pflastern mit einem Trägermaterial auf Polyurethanbasis.

Üblicherweise werden mehrschichtige dünne Pflaster mit einem Trägermaterial auf Polyurethanbasis dadurch hergestellt, daß die Polyurethanmasse auf einem Trägermaterial ausgestrichen wird und vor dem Aushärten mit einer Klebemasse, bevorzugt ebenfalls aus Polyurethan, von einem Rakel zusammenkaschiert wird.

Nicht möglich war es bisher, sehr dünne Trägerschichten aus Polyurethan herzustellen. Wird nämlich der Rakelspalt auf eine sehr geringe Höhe eingestellt, so kann zwar eine dünne Polyurethanschicht erzeugt werden, allerdings kommt es vor dem Rakel bedingt durch die hohe Viskosität des Polyurethans zu einer Ansammlung einer Masseflotte. Diese Masseflotte aus dem reaktiven Polyurethan verblockt im Laufe der Zeit, so daß ein kontinuierlicher Betrieb unmöglich ist.

US 5 935 363 A offenbart ein kontinuierliches Verfahren zur Herstellung von Pflastern bzw. Wundauflagen, bei dem zumindest eine Schicht eines Polyurethans auf einen Zwischenträger aufgebracht wird. Auf eine Trennlage wird eine Klebemasse aufgetragen. Anschließend werden das Polyurethan auf dem Zwischenträger und die Klebemasse auf der Trennlage in einem Walzenspalt zusammenkaschiert.

Am Beispiel von Schaumwundauflagen, wie sie zum Beispiel von der Firma Beiersdorf unter dem Namen Cutinova ® thin und Cutinova ® hydro erhältlich sind, soll wird der Herstellprozeß näher erläutert. U.a. beschrieben sind diese in DE 42 33 289 A1, in DE 196 18 825 A1 und WO 97/43328.

Danach besteht der Polyurethangelschaum aus einem Polyadditionsprodukt eines Polyetherpolyols (Levagel ® von Bayer AG) mit einem aromatischen oder aliphatischen Diisocyanat (Desmodur ® Bayer AG), in das ein Polyacrylat-Superabsorber-Pulver (Favor ®, Stockhausen) eingearbeitet wurde. Das Polyurethangel kann, je nach Verhältnis von OH-Äquivalenten des Polyols zu reaktiven Isocyanat-Gruppen, schwach oder stark selbsthaftend auf Haut eingestellt werden.

Der flächige Polyurethangelschaum mit einer Dicke von 1 bis 6 mm wird vor einem Rakel einseitig von einer Polyurethanfolie abgedeckt. Pflaster entsprechender Größe werden aus der Ballenware ausgestanzt.

Die ausgestanzten großflächigen Wundauflagen eignen sich hervorragend zur Versorgung von chronischen oder schwerheilenden Wunden von Patienten, die stationär versorgt werden müssen.

Aufgabe der Erfindung ist es, ein Verfahren zur Verfügung zu stellen, das in der Lage ist, sehr dünne, gegebenenfalls mehrschichtige Pflaster mit einem Polyurethanträger in einem kontinuierlichen Prozeß herzustellen.

Gelöst wird die Aufgabe durch ein Verfahren, wie es im Anspruch 1 dargelegt ist. Die Unteransprüche umfassen vorteilhafte Varianten des Erfindungsgegenstands.

Demgemäß betrifft die Erfindung ein Verfahren zur Herstellung von mehrschichtigen dünnen Pflastern mit einem Trägermaterial auf Polyurethanbasis, wobei

a) zumindest eine Schicht eines Polyurethans auf einen Zwischenträger aufgebracht wird,

b) auf eine Trennlage eine Klebemasse aufgetragen wird und

c) das nicht ausgehärtete Polyurethan auf dem Zwischenträger und die Klebmasse auf der Trennlage zusammenkaschiert werden, dadurch gekennzeichnet, dass

d) das Zusammenkaschieren durch ein Rakel erfolgt und

e) nach dem Rakel das Laminat in einen Walzenspalt geführt wird, in dem das Polyurethan sowie die Klebmasse auf die endgültige Dicke ausgewalzt werden.

In einer ersten vorteilhaften Ausführungsform des Verfahrens wird der Walzenspalt von zwei Glattwalzen gebildet.

Weiterhin bevorzugt ist, wenn der Walzenspalt von einer oberen Prägewalze und einer unteren Glattwalze gebildet wird.

Durch die Prägung ist es möglich, auf der laufenden Trägerbahn die Polyurethanschicht zum einen auf die gewünschte Dicke zu bringen und zum anderen in der geschlossenen Polyurethanschicht gezielte Materialansammlungen zu erzeugen, um somit beliebig geformte Pflaster ausstanzen zu können.

In der Prägewalze können verschiedene Einprägungen vorhanden sein, um eine Veränderung der Polyurethanschicht zu erzielen. Beispielsweise können diese die Form einer halbkonkaven Linse aufweisen. Dies führt in der Polyurethanschicht zu erhöhten Zentren, die sich zum Rand hin abschrägen. Möglich sind auch Ellipsoide, Quader, Würfel oder andere geometrische Formen, um spezielle Formen in der PU-Schicht zu erzeugen.

Beispielsweise ergibt sich bei der Linse eine Polyurethanschicht, die nach der Stanzung sich ausgehend von einem Punkt in der Mitte zum Rand hin abschrägt.

Insbesondere liegt dieser Punkt im Flächenzentrum, um ein symmetrisches Aussehen der sich abschrägenden Schicht zu erzielen. Die Abschrägung kann aber auch unregelmäßig erfolgen, je nach Bedarf und Anwendungsfall des Pflasters.

Die Höhe der Randzone beträgt maximal 50% von der Gesamthöhe des Produktes, bevorzugt werden Höhen der Randzone von kleiner 0,2 mm. Der Konturierungsverlauf vom Zentrum zum Rand wird durch die gewählte Form festgelegt, das heißt, die Gießform bestimmt das Design der Kontur.

Um die gewünschte Dickenreduzierung zu erreichen, wird im Walzenspalt vorzugsweise ein Druck von zumindest 4 bar, insbesondere 5 bar erzeugt.

Die Polyurethanschicht weist nach dem Walzenspalt weiter vorzugsweise eine Dicke von 10 bis 100 µm, insbesondere 40 bis 60 µm, auf.

Der Kleber weist nach dem Walzenspalt weiter vorzugsweise eine Dicke von 20 bis 100 µm, insbesondere 40 bis 60 µm, auf.

Die Trägerfolie besteht vorzugsweise aus einer transparenten mehrschichtigen wasserdampfdurchlässigen Polyurethan-, Polyethylen-, Polypropylen-, Polyamid- oder Polyester-Folie. Diese Aufzählung ist aber nicht abschließend zu verstehen, selbstverständlich kann der Fachmann ohne erfinderisches Zutun andere, geeignete Folien auffinden.

In einer vorteilhaften Ausführungsform weisen diese eine Dicke von 10 bis 100 µm, insbesondere 40 bis 60 µm, auf.

Die vorteilhafte Dicke resultiert aus der Anforderung nach einem biegesteifen Material, das das Aufrollen bei mechanischer Belastung unterbindet.

In einer weiteren vorteilhaften Ausführungsform besteht diese aus einer hautfreundlichen Haftklebemasse aus Polyacrylat, in die zur Erhöhung des Haftvermögens auf Haut ein Tackifier (zum Beispiel ein Kohlenwasserstoff-Harz) eingearbeitet wird.

Die Trennlage ist bevorzugt ein Trennpapier, das einseitig siliconisiert ist.

Die Polyurethanschicht ist insbesondere transparent, stark wasserdampfdurchlässig und klebend. Zur Speicherung von Flüssigkeit wird vorzugsweise ein Superabsorber-Polymer als Pulver eingearbeitet.

Die Polyurethanschicht dient als gute Polsterung und als Speicher von Exudat, das aus der Wunde austritt.

Geeignete Polyurethane sind Gegenstand der DE 196 18 825, in der hydrophile, selbstklebende Polyurethan-Gele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.-%,
b) Antioxidantien,
c) in den Polyolen a) löslichen Wismut-(HI)-Carboxylaten auf Basis von Carbonsäuren mit 2 bis 18 C-Atomen als Katalysatoren sowie
d) Hexamethylendiisocyanat,

mit einem Produkt der Funktionalitäten der Polyurethanbildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew.-%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥ 20 Gew.-%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.-% liegt.

Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)Salze verzweigter gesättigter Carbonsäuren mit tertiären Carboxylgruppen, wie der 2,2-Dimethyl- Octansäure (zum Beispiel Versatic-Säuren, Shell). Gut geeignet sind Zubereitungen dieser Bi (III) Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuß von 3 mol dieser Säure mit einem Bi-Gehalt von ca. 17%.

Es werden die Katalysatoren bevorzugt in Mengen von 0,03 bis 0,1 Gew.-%, bezogen auf das Polyol a), eingesetzt.

Als Antioxidantien kommen für die erfindungsgemäßen Polyurethan-Gele insbesondere sterisch gehinderte phenolische Stabilisatoren, wie BHT (2,6-Ditert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des α-Tocopherol eingesetzt.

Die Antioxidantien werden bevorzugt in Mengen von 0,15 bis 0,5 Gew.-%, bezogen auf das Polyol a), eingesetzt.

Die Isocyanatkennzahl (Verhältnis der bei der Reaktion eingesetzten freien NCO-Gruppen zu den freien OH-Gruppen) der erfindungsgemäßen Polyurethan-Gelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$f_{(Polyol)} \cdot (f_{(Isocyanat)} - 1) \cdot Kennzahl \approx 2$$

$$Kennzahl \approx \frac{2}{f_{(Polyol)} \cdot (f_{(Isocyanat)} - 1)}$$

f: Funktionalität der Isocyanat- oder Polyolkomponente

Je nach angestrebter Klebrigkeit oder Elastizität des Gels kann die tatsächlich zu verwendende Isocyanatkennzahl um bis zu + 20% von dem berechneten Wert abweichen.

Die erfindungsgemäßen Polyurethan-Gelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.

Weiter vorzugsweise kommen Polyurethane zum Einsatz, wie sie in der EP 0 665 856 B1 offenbart sind.

Die hydrophilen Polyurethangelschäume sind demnach erhältlich aus

    1. einem Polyurethangel, welches

        (A) 25-62 Gew.-%, vorzugsweise 30-60 Gew.-%, besonders bevorzugt 40-57 Gew.-%, bezogen auf die Summe aus (A) und (B), eines kovalent vemetzten Polyurethans als hochmolekulare Matrix und
        (B) 75-38 Gew.-%, vorzugsweise 70-40 Gew.-%, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

        (C) 0 bis 100 Gew.-%, bezogen auf die Summe aus (A) und (B), an Füll- und/oder Zusatzstoffen enthält,

und welches erhältlich ist durch Umsetzung einer Mischung von

        a) einem oder mehreren Polyisocyanaten,
        b) einer oder mehreren Polyhydroxylverbindungen mit einem mittleren Molekulargewicht zwischen 1000 und 12000, und einer mittleren OH-Zahl zwischen 20 und 112,
        c) gegebenenfalls Katalysatoren oder Beschleunigern für die Reaktion zwischen Isocyanat- und Hydroxylgruppen sowie gegebenenfalls
        d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen 3 und 6 beträgt und die Isocyanatkennzahl (K) der Formel

$$K = \frac{300 \pm X}{(F_I \cdot F_p) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

    2. einem Wasser absorbierenden Material und

    3. einem nichtwäßrigen Schäumungsmittel.

Die Polyurethangele können aus den an sich aus der Polyurethanchemie bekannten Ausgangsverbindungen nach an sich bekannten Verfahren hergestellt werden, wie sie zum Beispiel in DE 31 03 499 A1, DE 31 03 500 A1 und EP 0 147 588 A1 beschrieben werden. Wesentlich ist jedoch, daß bei der Auswahl der gelbbildenden Komponenten die oben definierten Bedingungen eingehalten werden, da sonst anstelle von selbsthaftenden Gelen klebfreie, elastische Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen.

Die Diisocyanate können insbesondere zum Beispiel aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Die Polyurethanmasse kann ungeschäumt, geschäumt, ungefüllt oder mit zusätzlichen Füllstoffen, wie beispielsweise Superabsorbern, Titandioxid, Zinkoxid, Weichmachern, Farbstoffen etc. eingesetzt werden. Für Anwendungen im Bereich transdermaler Pflastersysteme ist es auch möglich, die Polyurethanmasse in der zentralen Zone mit Wirkstoffen zu dotieren. Weiterhin können auch Hydrogele in halbfester bis fester Form mit aktiven Bestandteilen für die zentrale Zone verwendet werden.

Die Polyurethan-Gele können gegebenenfalls aus der Polyurethan-Chemie an sich bekannte Zusatzstoffe enthalten, wie zum Beispiel Füllstoffe und Kurzfasern auf anorganischer oder organischer Basis, Metallpigmente, oberflächenaktive Substanzen oder flüssige Streckmittel wie Substanzen mit einem Siedepunkt von über 150 °C. Als organische Füllstoffe seien beispielsweise Schwerspat, Kreide, Gips, Kieserit, Soda, Titandioxid, Ceroxid, Quarzsand, Kaolin, Russ und Mikrohohlkugeln genannt.

An organischen Füllstoffen können zum Beispiel Pulver auf Basis von Polystyrol, Polyvinylchlorid, Hamstoff-Formaldehyd und Polyhydrazodicarbonamid eingesetzt werden. Als Kurzfasern kommen zum Beispiel Glasfasern von 0,1 - 1 mm Länge oder Fasern organischer Herkunft, wie zum Beispiel Polyester- oder Polyamidfasern, in Frage. Metallpulver, wie zum Beispiel Eisen oder Kupferpulver, können ebenfalls bei der Gelbildung mitverwendet werden. Um den Gelen die gewünschte Färbung zu verleihen, können die bei der Einfärbung von Polyurethanen an sich bekannten Farbstoffe oder Farbpigmente auf organischer oder anorganischer Basis verwendet werden, wie zum Beispiel Eisenoxid- oder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE 37 13 601 A1 offenbart werden und auch Superabsorber der neuen Generation mit nur noch geringen Anteilen an austrockenbarem Wasser und hohem Quellvermögen unter Druck. Bevorzugte Produkte sind schwach vernetzte Polymerisate auf der Basis Acrylsäure/Natriumacrylat. Solche Natrium-polyacrylate sind als Favor 22-SK (Chemische Fabrik Stockhausen GmbH, Deutschland) erhältlich. Weitere Absorber, zum Beispiel Carboxymethylcellulose und Karaya, sind ebenfalls geeignet.

Der Schäumungsgrad läßt sich durch die eingearbeiteten Mengen an Schäumungsmittel in weiten Grenzen variieren.

Gegebenenfalls wird das selbstklebende Produkt durch ein silikonisiertes Papier oder eine insbesondere silikonisierte Folie abgedeckt, so daß die klebende Seite während der Lagerung geschützt ist.

Durch das erfindungsgemäße Verfahren ist es möglich, dünne PU-Pflaster mit einer Polyurethanmasse als Wundauflage in einem Arbeitsgang zu fertigen. Bei der bevorzugten Verfahrensvariante mit der geprägten Walze können sogar Produkte hergestellt werden, die sehr bis äußerst dünne Ränder aufweisen.

Bekannt ist bisher, Pflaster zu fertigen, deren Polyurethanmasseträger ein Flächengewicht von 120 g/m$^2$ bis 150 g/m$^2$ aufweist. Nunmehr lassen sich minimale Flächenaufträge von weniger als 30 g/m$^2$ erzeugen.

Im folgenden soll anhand eines Bildes ein besonders vorteilhaft ausgestaltetes, erfindungsgemäßes Verfahren zur Herstellung von mehrschichtigen dünnen Pflastern mit einem Trägermaterial auf Polyurethanbasis dargestellt werden.

Zunächst wird eine Schicht eines Polyurethans 11 auf einen Zwischenträger 12 aufgebracht. Gleichzeitig wird auf eine Trennlage 2 eine Klebemasse 3 aufgetragen. Dazu wird über eine Walze 5 die Trennlage 2 unter einen Behälter 4 gefahren, aus dem die Klebmasse 3 in einer gewissen Schichtdicke auf die Trennlage 2 aufgegeben wird.

Der Verbund 1 aus nicht ausgehärtetem Polyurethan 11 und Zwischenträger 12 wird mit der Klebmasse 3 auf der Trennlage 2 von einem Rakel 6 zusammenkaschiert und einem Walzenspalt zugeführt, der aus den beiden Walzen 7 und 8 gebildet wird.

In dem Walzenspalt werden das Polyurethan 11 sowie die Klebmasse 3 auf die endgültige Dicke ausgewalzt.
Der Walzenspalt kann dabei von zwei Glattwalzen 7, 8 gebildet werden, die obere Walze 7 kann aber auch eine Prägewalze sein.

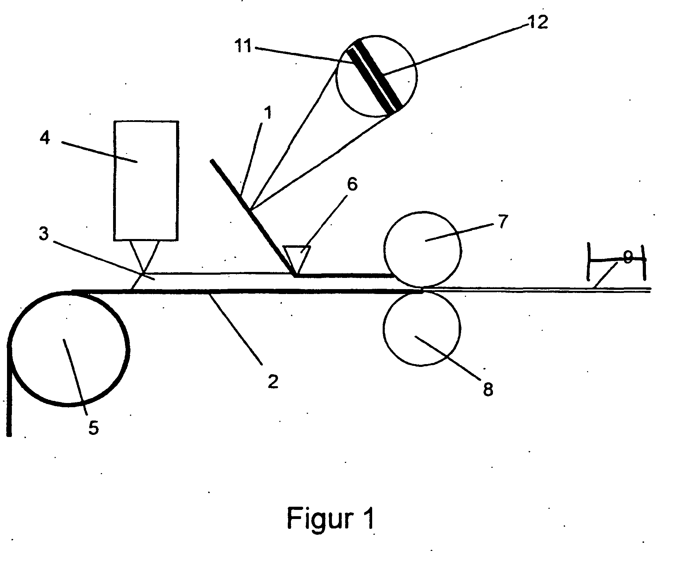

**Patentansprüche**

1.  Verfahren zur Herstellung von mehrschichtigen dünnen Pflastern mit einem Trägermaterial auf Polyurethanbasis, wobei

    a) zumindest eine Schicht eines Polyurethans auf einen Zwischenträger aufgebracht wird,
    b) auf eine Trennlage eine Klebemasse aufgetragen wird und
    c) das nicht ausgehärtete Polyurethan auf dem Zwischenträger und die Klebmasse auf der Trennlage zusammenkaschiert werden, **dadurch gekennzeichnet, dass**
    d) das Zusammenkaschieren durch ein Rakel erfolgt und
    e) nach dem Rakel das Laminat in einen Walzenspalt geführt wird, in dem das Polyurethan sowie die Klebmasse auf die endgültige Dicke ausgewalzt werden.

2.  Verfahren nach Anspruch 1, **dadurch gekennzeichnet, dass** der Walzenspalt von zwei Glattwalzen gebildet wird.

3.  Verfahren nach Anspruch 1, **dadurch gekennzeichnet, dass** der Walzenspalt von einer oberen Prägewalze und einer unteren Glattwalze gebildet wird.

4.  Verfahren nach zumindest einem der vorherigen Ansprüche, **dadurch gekennzeichnet, dass** im Walzenspalt ein Druck von zumindest 4 bar erzeugt wird.

5.  Verfahren nach Anspruch 4, **dadurch gekennzeichnet, dass** im Walzenspalt ein Druck von 5 bar erzeugt wird.

6.  Verfahren nach zumindest einem der vorherigen Ansprüche, **dadurch gekennzeichnet, dass** die Polyurethanschicht nach dem Walzenspalt eine Dicke von 10 bis 100 µm aufweist.

7.  Verfahren nach Anspruch 6, **dadurch gekennzeichnet, dass** die Polyurethanschicht nach dem Walzenspalt eine Dicke von 40 bis 60 µm aufweist.

8.  Verfahren nach zumindest einem der vorherigen Ansprüche, **dadurch gekennzeichnet, dass** der Kleber nach dem Walzenspalt eine Dicke von 20 bis 100 µm aufweist.

9.  Verfahren nach Anspruch 8, **dadurch gekennzeichnet, dass** der Kleber nach dem Walzenspalt eine Dicke von 40 bis 60 µm aufweist.

**Claims**

1.  Process for producing multilayered thin plasters comprising a carrier material based on polyurethane, which comprises

    a) applying at least one layer of a polyurethane to a transfer,
    b) applying an adhesive composition to a release layer,
    c) laminating the uncured polyurethane on the transfer and the adhesive composition on the release layer together **characterized in that**,
    d) the laminating is effected using a doctor blade and
    e) the laminate is passed downstream of the doctor blade into a roll nip in which the polyurethane and the adhesive composition are rolled out to the ultimate thickness.

2.  Process according to Claim 1, **characterized in that** the roll nip is formed by two smooth rolls.

3.  Process according to Claim 1, **characterized in that** the roll nip is formed by a top embossing roll and a smooth bottom roll.

4.  Process according to at least one preceding claim, **characterized in that** the roll nip generates a pressure of at least 4 bar.

5.  Process according to Claim 4, **characterized in that** the roll nip generates a pressure of 5 bar.

6.  Process according to at least one preceding claim, **characterized in that** the polyurethane layer has a post roll nip thickness of 10 to 100 µm.

7.  Process according to Claim 6, **characterized in that** the polyurethane layer has a post roll nip thickness of 40 to 60 µm.

8.  Process according to at least one preceding claim, **characterized in that** the adhesive has a post roll nip thickness of 20 to 100 µm.

9.  Process according to Claim 8, **characterized in that** the adhesive has a post roll nip thickness of 40 to 60 µm.

**Revendications**

1. Procédé pour la fabrication de pansements minces à plusieurs couches comprenant un matériau support à base de polyuréthane, dans lequel

   a) on applique au moins une couche d'un polyuréthane sur un support intermédiaire,
   b) on applique une masse adhésive sur une couche de séparation et
   c) le polyuréthane non durci sur le support intermédiaire et la masse adhésive sur la couche de séparation sont contrecollés, **caractérisé**
   d) en ce que le contrecollage est réalisé au moyen d'une racle
   e) en ce que le produit laminé, après la racle, est guidé dans une fente de laminage dans laquelle le polyuréthane ainsi que la masse adhésive sont laminés à l'épaisseur finale.

2. Procédé selon la revendication 1, **caractérisé en ce que** la fente de laminage est formée par deux cylindres lisses.

3. Procédé selon la revendication 1, **caractérisé en ce que** la fente de laminage est formée par un cylindre de moletage supérieur et un cylindre lisse inférieur.

4. Procédé selon au moins l'une quelconque des revendications précédentes, **caractérisé en ce qu'**on produit une pression d'au moins 4 bars dans la fente de laminage.

5. Procédé selon la revendication 4, **caractérisé en ce qu'**on produit une pression de 5 bars dans la fente de laminage.

6. Procédé selon au moins l'une quelconque des revendications précédentes, **caractérisé en ce que** la couche de polyuréthane présente une épaisseur de 10 à 100 μm en aval de la fente de laminage.

7. Procédé selon la revendication 6, **caractérisé en ce que** la couche de polyuréthane présente une épaisseur de 40 à 60 μm en aval de la fente de laminage.

8. Procédé selon au moins l'une quelconque des revendications précédentes, **caractérisé en ce que** la colle présente une épaisseur de 20 à 100 μm en aval de la fente de laminage.

9. Procédé selon la revendication 8, **caractérisé en ce que** la colle présente une épaisseur de 40 à 60 μm en aval de la fente de laminage.

Figur 1